# EUROPEAN PATENT APPLICATION

(11) **EP 2 022 533 A1**
(43) Date of publication of application: **11.02.2009**
(21) Application number: 07015120.4
(22) Date of filing: 01.08.2007
(51) Int. Cl.: A61N 5/06

(54) **Induction driven light module and use thereof**

(71) Applicant: National Yang-Ming University, Beitou District Taipei City 112 (TW)
(72) Inventor: Kao, Fu-Jen c/o National Yang-Ming University, Taipei City 112, Taiwan (CN); Yang, Chyun-Yu, Tainan 704, Taiwan (CN); Li, Cheng-Chun c/o National Yang-Ming University, Taipei City 112, Taiwan (CN)
(74) Representative: TER MEER - STEINMEISTER & PARTNER GbR

(57) **Abstract**

The present invention relates to an induction driven light module. In particular, the present invention is directed to an induction driven LED (20,30) or LD (10) module for photodynamic therapy, entertainment or decoration. The present invention also relates to a method for treating cancer, tumor or a specific ailment.

## Description

### FIELD OF THE INVENTION

The present invention relates to an induction driven light module. In particular, the present invention is directed to an induction driven LED or LD module for photodynamic therapy, entertainment or decoration use. The present invention also relates to a method for treating cancer, tumor or a specific ailment.

### BACKGROUND OF THE INVENTION

### PDT

Photodynamic therapy (PDT) has been proposed as one alternative for treating prostatic tissue. U.S. Pat. No. 5,514,669 (Selman) describes a method of treating the symptoms associated with BPH or prostatitis comprising sensitizing the prostatic tissue with an effective amount of photosensitive composition which accumulates in the tissue and exposing the sensitized tissue to a light source whereby the photosensitive composition absorbs the light or undergoes a photochemical reaction.

PDT or photodynamic therapy has become a recognized means of treating certain types of cancer. In essence, a photosensitizer such as PHOTOFRIN@PHOTOFRINsPHOTOFRIN@PHOTOFRINs porfimer sodium or BPD (benzoporphyrin derivative) is administered systemically to a patient with cancer or other ailments. The ideal photosensitizer distributes through the body in such a manner that it is highly concentrated at the diseased site. This process can take several hours to a few days depending on the pharmaceutical dynamics of the drug. A suitable light source is then used to activate the drug in the targeted tissue.

A common alternative to the above scenario is to administer the drug topically to the target tissue, e.g., a psoriatic lesion, site of viral infection, wart, or port wine stain. Once again, after the drug has been taken up by the target site, the light source is directed toward the target to activate the drug photochemically, either via a fiber or by direct illumination.

Several photosensitizing compounds have been tested in vivo as potential clinical photosensitizing drugs, including PHOTOFRIN* and its precursor hematoporphyrin derivative, BPD, chloroaluminum phthalocyanine tetrasulfonate, zinc phthalocyanine tetrasulfonate, protoporphyrin IX, purpurin, merocyanine 540, methylene blue, tetraphenylporphyrin sulfonate, pheophorbide, monoaspartyl chlorine. These photosensitizers can be activated by light in the 500 nm to 780 nm range.

The mechanisms of PDT are rather complicated, and the activation mechanisms may differ from one photosensitizer to another. However, a common feature of all these photosensitizers is that they are activated by the absorption of light. For effective absorption, the wavelength of excitation light must coincide with the photosensitizer's absorption band. The absorption results in the photo-excitation of the photosensitizer and subsequently initiates a series of chemical reactions, for example, rapid free radical generation, which then result in the death or destruction of the targeted cells or tissues.

Since the activation only takes place at where the photosensitizer located, only cells local to the photosensitizer are killed. The mechanism of the cell death depends on the treatment performed or drug used. For example, in the case of PHOTOFRIN, the tumor killing process appears to give rise to both localized destruction of the tumor tissues and to local vascular damage to the blood vessels supplying the tumor.

The net result for correct PDT treatment is to eliminate the tumor while keeping the the surrounding healthy tissues intact that absorbs relatively fewer photosensitizers. To optimize cancer cell killing, the photosensitizer should have a high absorption cross section, and the energy absorbed by photosensitizer should be efficiently converted into cancer cell killing chemical reaction. One mechanism by which this is achieved is outlined here. A photosensitizer in a singlet ground state is photo-excited into an excited singlet electronic state by photon absorption. This absorption will be strong since it is a dipole allowed transition.

### Light Sources for PDT

From a practical point of view, LEDs and LDs have a number of advantages. They operate at low voltages (∼ usually less than 3V) depending on the bandgap of the materials, and consumes small amount of current (in the order of milliamp). They can be packaged into systems that are powered either from a wall socket or a battery pack. The low voltage allows easy compliance with electrical safety and medical requirements: they can be easily carried or wheeled around on a small trolley, or assembled into shapes or sizes appropriate for specialized applications. The monolithic structure of the LEDs makes it simple to design systems that can satisfy the requirements for sterility in a hospital environment. It is possible to connect LEDs together in a variety of ways so that the voltage-current requirements are tailored to the electrical power supply that is available. In short, LEDs and LDs are efficient and versatile light sources.

Recently, light-emitting diodes (LEDs) have become commercially important illumination sources for high-luminance applications. LEDs are miniature semiconductor light sources installed as a thin, active layer within a block of partially transparent material with high index of refraction. For example, Aluminum Indium Gallium Phosphide has optical index of refraction 3.6, while Indium Gallium Nitride has index of 2.4. LEDs typically employ a transparent encapsulation, often of epoxy, with refractive index approximately 1.5. This increases the emission fraction to one fourth, and reduces Fresnel reflectance to one ninth (ironically, swapping their values). The two most commercially significant encapsulating geometries are the bullet shaped lens and the globbed die-on-board (i.e. encapsulation covering the die mounted on a circuit board). In both cases, however, light that does escape the die must undergo further light trapping of about 50%, offsetting the full improvement possible. The losses of the bullet lens are inherent in its convenient shape, presently manufactured by the billions.

### Induction Coupling of Energy

In this invention, inductive driving will be used as described in the U.S. Pat. No. 5,193,539, "Implantable Microstimulator". This referred patent describes a microstimulator in which power and information for operating the microstimulator are received by an induction coil through a modulated, alternating magnetic field. The induction coil receives energy from outside the patient's body and a capacitor is used to store electrical energy which is released to the microstimulator's exposed electrodes under the control of electronic control circuitry.

### Related Patents and References

Photodynamic therapy involves the administration of a photosensitising drug to an affected area, and its subsequent irradiation with light.

The document GB 2,212,010 discloses a therapeutic light source which uses an array of discrete LED's as an alternative to lasers or laser diodes. The output of the LED's is focussed so as to provide the necessary intensity.

WO 94/15666 discloses a therapeutic light source specifically for PDT, with an integrated array of LED's mounted on the distal end of a hand piece. The LED's are overdriven to give the necessary intensity, and cooled by the flow of water around a closed loop passing along the hand piece.

U.S. Pat. No. 5,728,090 discloses a somewhat similar device with various different types of head containing integrated LED matrices. These devices require complicated liquid cooling circuits which would add to the cost of the device and add to the bulk of the hand piece, which is disadvantageous for invasive use.

U.S. Pat. No. 5,728,090 mentions that the wavelength of the LED's is between 300 nm and 1300 nm and is selected based upon the particular photosensitive dye used during PDT. However, the wavelengths of LED's capable of providing the necessary intensity for PDT cannot freely be chosen within that range.

### SUMMARY OF THE INVENTION

The present invention relates to an induction driven light module comprising (a) at least one light generating device operably linked or coupled to a driver induced by alternating magnetic field, and (b) a packaging material to cover or seal the component (a).

The present invention also relates to a method for treating a subject suffering cancer, tumor, or selected tissues comprising (a) implanting the induction driven light module of the present invention into a tissue nearby or adjacent to cancer, tumor or selected tissues of the subject; (b) administering a photosenstizer on cancer, tumor or selected tissues; (c) driving the light module through the induction of alternating magnetic field; and (d) irradiating cancer, tumor, or selected tissues by the driven light generating device.

The present invention further relates to a method of treating angiogenesis in a subject comprising (a) implanting the induction driven light module of the present invention into a tissue nearby or adjacent to blood vessel of the subject; (b) administering a photosenstizer on cancer, tumor or selected tissues; (c) driving the light module through the induction of alternating magnetic field; and (d) irradiating cancer, tumor, or selected tissues by the driven light generating device.

### BRIEF DESCRIPTION OF THE DRAWINGS

Figure 1 illustrates one embodiment of the induction driven light module of the present invention.
Figure 2 illustrates another embodiment of the induction driven light module of the present invention.

### DETAILED DESCRIPTION OF THE INVENTION

Accordingly, the present invention provides an induction driven light module comprising (a) at least one light generating device operably linked or coupled to a driver induced by alternating magnetic field, and (b) a packaging material to cover or seal the component (a).

An induction module consists of coils of insulated copper wire wound around iron cores. Because of the large number of turns in the coil, the induction voltage is enhanced sufficiently to drive the light source. The term "*Induction coil*" is also used for a coil carrying high-frequency AC and intended to induce eddy currents to heat objects placed in the interior of the coil, such as in induction heating or zone melting, representing another example based on the Faraday induction law.

In the present invention, the light source is not limited but includes a light-emitting diode or a laser diode.

In a preferred embodiment, the light-emitting diode or laser diode provides low irradiation power or high irradiation power.

In the present invention, the driver is powered by a broad band of frequencies (from low to high) of alternating magnetic field or RF power with frequencies that can easily penetrate human body. In a preferred embodiment, the driver is induced by low frequency alternating magnetic field or RF power with frequencies that can easily penetrate human body.

To avoid direct contact of electrodes and tissues, the module of the present invention is sealed with a water-tight material. It is also intended that a bio-compatible water-tight packaging is implemented to enable the module of the present invention to be cleaned without risking damaging the electrical contacts. To enable water-tight capacity and safety consideration, the module of the present invention can be covered or sealed from a plastic, examples of which include but are not limited to polyurethane, polyester, polycarbonate, polylactic acid, polyglycolic acid, poly(lactide-co-glycolide), poly(.epsilon.-caprolactone), polyethyleneimine, polystyrene, polyamide, rubber, nylon, TEFLON, silicone rubber, polyacrylonitrile, polyacrylate, and polymetacrylate, poly(alpha-hydroxy acid), poly(dioxanone), poly(orthoester), poly(ether-ester), poly(lactone), polytetrafluoroethylene, organosilane, mixtures thereof and copolymers thereof. In a preferred embodiment, the material is biocompatible and selected from the group consisting of polyurethane, polyester and polycarbonate. In a further preferred embodiment, the material is semi-transparent or transparent. In more preferred embodiment, the material covering or sealing the light generating device is transparent.

### Entertainment, aesthetics, architectural and decorative applications and articles

The induction driven light module of the present invention provides numerous color changing possibilities to enhance our environment. The infinite color palette ranging from pretty pastels to deep intense hues makes these products ideal for entertainment, aesthetics, architectural and decorative applications.

Using current color mixing devices, the induction driven light module of the present invention gives the architect a full coverage of color palette in a range of lighting fixtures that can enhance building facades, water features and focal displays.

By combining programmable alternating magnetic field and control system for producing multi-colored lighting effects, the induction driven light module of the present invention can generates extraordinary effects, provides a canvas for creative lighting designs, effects, animation or colored accents.

Techniques are known for producing multi-colored lighting effects with LED's. Some such techniques are shown in, for example, U.S. Pat. No. 6,016,038, U.S. patent application Ser. No. 09/215,624, and U.S. Pat. No. 6,150,774, the teachings of which are incorporated herein by reference. While these references demonstrate systems for producing lighting effects, they do not address some applications of programmable, multi-colored lighting systems.

For example, many toys, such as balls, benefit from improved color illumination processing, and/or networking attributes. There are toy balls that have lighted parts or balls where the entire surface appears to glow; however there is no ball available that employs dynamic color changing effects. Moreover, there is no ball available that responds to data signals provided from a remote source. As another example, ornamental devices are often lit to provide enhanced decorative effects. U.S. Pat. Nos. 6,086,222 and 5,975,717, for example, disclose lighted ornamental icicles with cascading lighted effects. As a significant disadvantage, these systems apply complicated wiring harnesses to achieve dynamic lighting. Other examples of crude dynamic lighting may be found in consumer products ranging from consumer electronics to home illumination (such as night lights) to toys to clothing, and so on.

### Medical Applications

The present invention also provides a method for treating a subject suffering cancer, tumor, or selected tissues comprising (a) implanting the induction driven light module of the present invention into a tissue nearby or adjacent to cancer, tumor or selected tissues of the subject; (b) administering a photosenstizer on cancer, tumor or selected tissues; (c) driving the light module through the induction of alternating magnetic field; and (d) irradiating cancer, tumor, or selected tissues by the driven light generating device.

In the present method, the targeted tissues are not limited but include breast, brain, liver, kidney, stomach, pancreas, intestine, spleen, bone marrow, joints, heart, lung or body parts that are difficult to be accessed with endoscopy. In a preferred embodiment, the issue is breast, brain, liver, kidney, bone marrow, joints, heart or lung.

The subject mentioned herein is not limited to mammals or birds. In a preferred embodiment, the subject is a human.

In the method of the present invention, the irradiation can be long term or short term.

As used herein, "photosensitizer" or "photosensitizing agent" means a chemical compound which, when absorbs light radiation, induces changes to, or destruction of, the prostatic tissue. Preferably, the chemical compound is nontoxic to humans or is capable of being formulated in a nontoxic composition. Preferably, the chemical compound in its photodegraded form is also nontoxic. The invention may be practiced with a variety of synthetic and naturally occurring photosensitizers, including, but not limited to, pro-drugs such as the pro-porphyrin 5-aminolevulinic acid (ALA) and derivatives thereof, porphyrins and porphyrin derivatives e.g. chlorins, bacteriochlorins, isobacteriochlorins, phthalocyanine and naphthalocyanines and other tetra- and poly-macrocyclic compounds, and related compounds (e.g. pyropheophorbides, sapphyrins and texaphyrins) and metal complexes (such as, but not limited by, tin, aluminum, zinc, lutetium). Tetrahydrochlorins, purpurins, porphycenes, and phenothiaziniums are also within the scope of the invention. Other suitable photosensitizers include bacteriochlorophyll derivatives such as those described in WO-A-97/19081, WO-A-99/45382 and WO-A-01/40232. A preferred bacteriochlorophyll is palladium-bacteriopheophorbide WST09 (Tookad.TM.). Preferably the photosensitizers are selected from pro-porphyrins, porphyrins, and mixtures thereof. Some examples of pro-drugs include aminolevulinic acid such as Levulan.TM. and aminolevulinic acid esters such as described in WO-A-02/10120 and available as Metvix.TM., Hexvix.TM. and Benzvix.TM.. Some examples of di-hydro or tetra-hydro porphyrins are described in EP-A-337,601 or WO-A-01/66550 and available as Foscan.TM. (temoporfin).

In preferred embodiments of the invention, the photosensitizer is selected from a particularly potent group of photosensitizers known as green porphyrins, which are described in detail in U.S. Pat. No. 5,171,749 (incorporated herein in references). The term "green porphyrins" refers to porphyrin derivatives obtained by reacting a porphyrin nucleus with an alkyne in a Diels-Alder type reaction to obtain a mono-hydrobenzoporphyrin. Such resultant macropyrrolic compounds are called benzoporphyrin derivatives (BPDs), which is a synthetic chlorin-like porphyrin with various structural analogues, as shown in U.S. Pat. No. 5,171,749. Typically, green porphyrins are selected from a group of tetrapyrrolic porphyrin derivatives obtained by Diels-Alder reactions of acetylene derivatives with protoporphyrin under conditions that promote reaction at only one of the two available conjugated, nonaromatic diene structures present in the protoporphyrin-IX ring systems (rings A and B). Metallated forms of a Gp, in which a metal cation replaces one or two hydrogens in the center of the ring system, may also be used in the practice of the invention. The preparation of the green porphyrin compounds useful in this invention is described in detail in U.S. Pat. No. 5,095,030 (hereby incorporated by reference).

Additionally, the photosensitizers used in the invention may be conjugated to various ligands to facilitate targeting. These ligands include receptor-specific peptides and/orc ligands as well as immunoglobulins and fragments thereof. Preferred ligands include antibodies in general and monoclonal antibodies, as well as immunologically reactive fragments of both.

Dimeric forms of the green porphyrin and dimeric or multimeric forms of green porphyrin/porphyrin combinations can be used. The dimers and oligomeric compounds of the invention can be prepared using reactions analogous to those for dimerization and oligomerization of porphyrins per se. The green porphyrins or green porphyrin/porphyrin linkages can be made directly, or porphyrins may be coupled, followed by a Diels-Alder reaction of either or both terminal porphyrins to convert them to the corresponding green porphyrins. Of course combinations of two or more photosensitizers may be used in the practice of the invention.

In addition to the above mentioned preferred photosensitizing agents, other examples of photosensitizers useful in the invention include, but are not limited to, green porphyrins disclosed in U.S. Pat. Nos. 5,283,255, 4,920,143, 4,883,790, 5,095,030, and 5,171,749; and green porphyrin derivatives, discussed in U.S. Pat. Nos. 5,880,145 and 5,990,149. Several structures of typical green porphyrins are shown in the above cited patents, which also provide details for the production of the compounds.

The present invention further provides a method of treating angiogenesis in a subject comprising (a) implanting the induction driven light module of the present invention into a tissue nearby or adjacent to blood vessel of the subject; (b) administering a photosenstizer on cancer, tumor or selected tissues; (c) driving the light module through the induction of alternating magnetic field; and (d) irradiating cancer, tumor or selected tissues by the driven light generating device.

In the method of the present invention, the treatment is not limited but includes cavernous hemangioma or hemangioendothelioma.

### EXAMPLES

### Example 1: Preparation of module of present invention

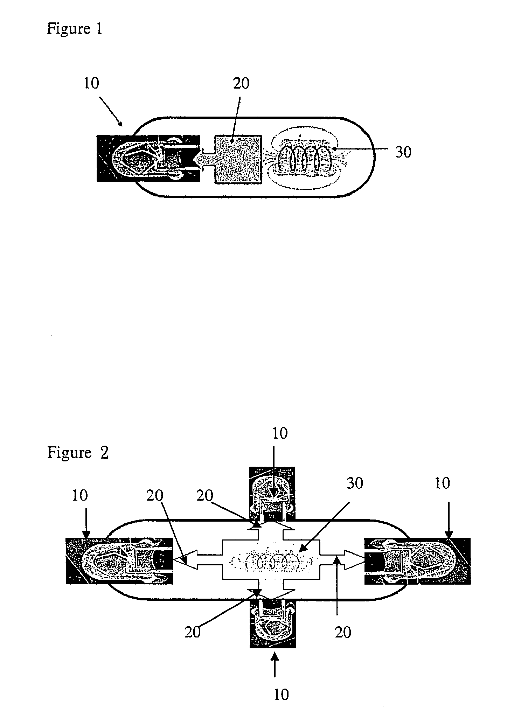

Referring now to FIG 1, a light generating device 10 (such as LED) was electrically coupled to a driver 20 having coil module 30 to form the module of the present invention. To avoid contacting the tissue, the use of a water-tight material such as polyurethane or polyester to seal the module of the present invention.

### Example 2: Application of present module to patient suffering cancer

A patient suffering liver cancer was selected to perform photodynamic therapy. The tissue nearby hepatoma was incised by surgery, was coated with photofrin and was placed with the module of the present invention. Then, the wound was closed and LED started to irradiate the hepatoma through inducing the coil based driver by alternating magnetic field.

It is understood that the following embodiments of the present invention are intended to be illustrative of some of the possible applications or principles. Various modifications may be made by the skilled person without departing from the true spirit and scope of the invention.

## Claims

1. An induction driven light module comprising (a) at least one light generating device operably linked or coupled to a driver induced by alternating magnetic field, and (b) a packaging material to cover or seal the component (a).

2. The module of Claim 1 wherein the light generating device is a light-emitting diode or a laser diode.

3. The module of Claim 2 wherein the light-emitting diode or laser diode provides low or high irradiation power.

4. The module of Claim 1 wherein the driver is induced by low frequency alternating magnetic field or RF power with frequencies that can easily penetrate human body.

5. The module of Claim 1, wherein the packaging material is selected from the group consisting of polyurethane, polyester, polycarbonate, polylactic acid, polyglycolic acid, poly(lactide-co-glycolide), poly(.epsilon.-caprolactone), polyethyleneimine, polystyrene, polyamide, rubber, nylon, TEFLON, silicone rubber, polyacrylonitrile, polyacrylate, and polymetacrylate, poly(alpha-hydroxy acid), poly(dioxanone), poly(orthoester), poly(ether-ester), poly(lactone), polytetrafluoroethylene, organosilane, mixtures thereof and copolymers thereof.

6. The module of Claim 5, wherein the packaging material is a material selected from the group consisting of polyurethane, polyester and polycarbonate.

7. The module of Claim 6, wherein the packaging material is semi-transparent or transparent.

8. The module of Claim 1, which is applied to entertainment, decoration or colored light generation.

9. The module of Claim 1, which is applied to articles for entertainment, decoration or colored light generation.

10. A method for treating a subject suffering cancer, tumor, or selected tissues comprising (a) implanting the induction driven light module of Claim 1 into a tissue nearby or adjacent to cancer, tumor or selected tissues of the subject; (b) administering a photosenstizer on cancer, tumor or selected tissues; (c) driving the light module through the induction of alternating magnetic field; and (d) irradiating cancer, tumor, or selected tissues by the driven light generating device.

11. The method of Claim 10 wherein the tissue is breast, brain, liver, kidney, stomach, pancreas, intestine, spleen, bone marrow, joints, heart, lung or body parts that are difficult to be accessed with endoscopy.

12. The method of Claim 10 wherein the light is originated from a light-emitting diode or a laser diode.

13. The method of Claim 12 wherein the light-emitting diode or laser diode provides low irradiation power.

14. The method of Claim 12 wherein the light-emitting diode or laser diode provides high irradiation power.

15. The method of Claim 10 wherein the driving the light module is induced by low frequency alternating magnetic field or RF power with frequencies that can easily penetrate human body.

16. The method of Claim 13 wherein the irradiation is long term.

17. The method of Claim 14 wherein the irradiation is short term.

18. A method of treating angiogenesis in a subject comprising (a) implanting the induction driven light module of Claim 1 into a tissue nearby or adjacent to blood vessel of the subject; (b) administering a photosenstizer on cancer, tumor or selected tissues; (c) driving the light module through inducing of alternating magnetic field; and (d) irradiating cancer, tumor or selected tissues by the driven light generating device.

19. The method of Claim 18 wherein the light is a light-emitting diode or a laser diode.

20. The method of Claim 18 wherein the treatment is directed to cavernous hemangioma or hemangioendothelioma.
